# EUROPÄISCHE PATENTSCHRIFT

(11) **EP 1 555 933 B1**
(45) Veröffentlichungstag und Bekanntmachung des Hinweises auf die Patenterteilung: **07.10.2009**
(21) Anmeldenummer: 03750231.7
(22) Anmeldetag: 22.10.2003
(51) Int. Cl.: A61B 5/00, G01N 11/16, G01N 11/14

(54) **SENSORSYSTEM FÜR DIE BESTIMMUNG DER GLUKOSE-KONZENTRATION IM BLUT**
SENSOR SYSTEM FOR DETERMINING THE CONCENTRATION OF GLUCOSE IN BLOOD
SYSTEME CAPTEUR DESTINE A LA DETERMINATION DE LA GLYCEMIE

(30) Priorität: 28.10.2002 EP 02024022; 16.05.2003 CH 887032003
(43) Veröffentlichungstag der Anmeldung: 27.07.2005
(73) Patentinhaber: Sensile Pat AG, 6340 Baar (CH)
(72) Erfinder: STRÄSSLER, Sigfrid, CH-1113 St-Saphorin-sur-Morges (CH); RYSER, Peter, CH-1110 Morges (CH); GANZ, Klaus, CH-8700 Küsnacht (CH); JACOT, Jacques, CH-2046 Fontaines (CH)
(74) Vertreter: Reuteler, Raymond Werner
(86) Internationale Anmeldenummer: PCT/CH2003/000684
(87) Internationale Veröffentlichungsnummer: WO 2004/037079

(56) Entgegenhaltungen:
- EP-A- 0 554 955
- DE-A- 3 714 708
- GB-A- 2 335 496
- US-A- 5 494 639
- US-A1- 2001 045 122
- US-B1- 6 201 980
- US-B1- 6 210 326
- US-B1- 6 271 044

## Beschreibung

Die Erfindung liegt auf dem Gebiet der Blutzucker-Messung durch einen implantierbaren Sensor. Diabetes mellitus (Zuckerkrankheit) ist eine der häufigsten chronischen Erkrankungen. Etwa 8% der US Bevölkerung sind betroffen und infolge zunehmendem Uebergewicht in der Bevölkerung steigt diese Zahl jährlich. Man rechnet weltweit mit gegen 300 Millionen Diabetikern bis im Jahre 2025. Eine langjährige und ungenügend behandelte Zuckerkrankheit bedeutet hohes Risiko für Herzinfarkt, Hirnschlag, Durchblutungsstörungen der unteren Extremitäten, Nierenschaden, Blindheit sowie Nervenleitungsstörungen, welche zu Amputationen der Füsse oder Beine führen können. Der Diabetes belastet deshalb das Gesundheitswesen mit ca. 10% aller Kosten.

Durch verschiedene Studien wie der Diabetes Control and Complication Trial (DCCT) und die U. K. Prospective Diabetes Study (UKPDS) konnte gezeigt werden, dass durch eine verbesserte Blutzuckereinstellung das Risiko von Spätkomplikationen vermindert werden kann. Zur Blutzuckersenkung stehen verschiedene Behandlungsarten zur Verfügung: eine angepasste Ernährung, körperliche Betätigung, Tabletten und Insulin. Ein wesentliches Element, die Effizienz der jeweiligen Behandlung zu überprüfen ist die Blutzucker-Selbstmessung (Self- monitoring). Alle insulinabhängigen Diabetiker (Typ 1) und eine Auswahl von nicht insulin- abhängigen Diabetikern (Typ 2) sollten mehrmals täglich ihren Blutzucker messen. Bis heute geschieht dies durch einen Stich an der Fingerkuppe und Auftragen einer kleinen Blutmenge auf einen Teststreifen, welcher in ein Ablesegerät geschoben wird. Diese Art von Selbstmessung ist mit Schmerzen und hohen Kosten verbunden. Seit Jahren wird daher ein schmerzloses Verfahren zur kontinuierlichen Blutzuckermessung angestrebt. Möglichst viele Blutzuckermessungen sollten den Aerzten und Patienten erlauben, die Behandlungen laufend anzupassen und zu verbessern, wodurch das Risiko von Spätkomplikationen verringert und die Folgekosten gesenkt werden könnten.

Die vorliegende Erfindung betrifft ein Sensorsystem für die Bestimmung der Glukose-Konzentration im Blut, mit einem implantierbaren Sensor und einem diesem zugeordneten Bediengerät.

Dokument GB-A-2 335 496 beschreibt ein Sensorsystem für die Bestimmung einer Glukose-Konzentration im Blut gemäß der Präambel der Ansprüche 1 und 10. Das Sensorsystem beinhaltet insbesondere einen implantierbaren Fühler und eine Abfrageeinheit. Der Fühler beinhaltet eine Hülle aus semipermeablem Material, darin eingeschlossen ist makromolekulares Material, welches einen Sensor, einen Schaltkreis, sowie ein Antwortsendegerät umspült.

Es ist ein transcutanes System mit einem implantierbaren Sensor bekannt, der eine Nadel aufweist, welche zwei durch einen Isolator getrennte verschiedene Metalle enthält, so dass ein elektrisches Potential angelegt werden kann. Der Sensor ist mit einem Monitor verbunden, der alle 5 Minuten über maximal 3 Tage die Glukosewerte aufzeichnet. Der Sensor ist nicht sehr stabil, so dass mehrmals täglich eine Eichung mit dem Blut des Patienten vorgenommen werden muss.

Bei einem anderen heute auf dem Markt erhältlichen Messsystem zur Messung des Glukosegehalts wird durch Stromimpulse Glukose durch die Haut gezogen und in zwei Gel-Scheiben eines Sensors gesammelt, welcher den Glukosegehalt misst. Der Sensor, der auf der Rückseite eines uhrartigen Anzeigegeräts angeordnet ist, ist ein so genanntes minimal invasives System, das ist ein System, bei welchem man entweder etwas auf die Haut auftragen oder kleine Kanülen in diese stechen muss, wodurch ein Infektionsrisiko nicht ausgeschlossen werden kann. Aus diesem Grund muss bei diesem invasiven System der Sensor alle paar Tage gewechselt werden, ausserdem erfordert auch dieses System eine Eichung mit dem Blut des Patienten. Beide genannten bekannten Systeme werden auch als Holtersysteme bezeichnet, darunter versteht man Systeme für den Gebrauch durch einen Arzt und nicht durch den Patienten selbst.

Durch die Erfindung soll ein Sensorsystem angegeben werden, welches für den Gebrauch durch den Patienten geeignet ist und diesem eine ständige Überwachung des Glukosegehalts seines Blutes ermöglichen soll, ohne dass nach der Implantation des Sensors schon nach kurzer Zeit ein neuerlicher Eingriff erforderlich ist, oder ein Infektionsrisiko darstellende Manipulationen an oder in der Haut des Patienten erforderlich sind.

Die gestellte Aufgabe wird erfindungsgemäss dadurch gelöst, dass der Sensor die Form einer Ampulle aufweist, in welcher eine sensitive Flüssigkeit eingeschlossen ist und in welche Glukose eindringen kann, dass eine Messung der Viskosität des aus der sensitiven Flüssigkeit und der Glukose bestehenden Gemisches erfolgt, und dass das Bediengerät durch ein aussen auf der Haut zu tragendes, portables Gerät gebildet ist, wobei die Steuerung der Messung und deren Auswertung durch das Bediengerät erfolgt.

Beim erfindungsgemässen Sensorsystem erfolgt durch das Bediengerät keinerlei Manipulation an oder in der Haut, so dass jede Reiz- und Infektionsgefahr ausgeschlossen ist. Der Sensor kann zumindest mehrere Monate lang implantiert sein, ohne dass eine Nacheichung oder dergleichen erforderlich wäre und dem Patienten bleiben die lästigen Blutabnahmen erspart. Der Patient kann jederzeit ohne irgendwelche Beschwerden den Glukosegehalt seines Blutes prüfen und diesen durch Einnahme entsprechender Medikamente regulieren, ohne dass eine Überwachung durch einen Arzt erforderlich wäre.

Eine erste bevorzugte Ausführungsform des erfindungsgemässen Sensorsystems ist dadurch gekennzeichnet, dass die Messung der Viskosität anhand des Schwingverhaltens eines im Sensor angeordneten Schwingorgans erfolgt, welches durch ein oszillierendes Magnetfeld zu Schwingungen anregbar ist, wobei des Schwingverhalten des Schwingorgans anhand von dessen Abklingverhalten nach Abschalten des Magneten analysiert wird. Das Schwingorgan selbst erzeugt ein Magnetfeld, welches vom Bediengerät gemessen wird. Vorteilhafte Weiterbildungen dieser ersten bevorzugten Ausführungsform des erfindungsgemässen Sensorsystems sind in den Ansprüchen 2 bis 9 beansprucht.

Eine zweite bevorzugte Ausführungsform des erfindungsgemässen Sensorsystems ist dadurch gekennzeichnet, dass die Messung der Viskosität anhand der Rotation eines im Sensor angeordneten Messorgans erfolgt, welches von einem ebenfalls im Sensor angeordneten Antriebsmagneten antreibbar ist, wobei die Rotation des Messorgans anhand von deren Abklinghalten nach Abschalten des Antriebsmagneten analysiert wird.

Vorzugsweise ist der Sensor zweistufig aufgebaut und weist ein Kopfteil und ein Messteil auf, wobei das Kopfteil den Antriebsmagneten und das Messteil das Messorgan enthält und der Antriebsmagnet gegen Flüssigkeit abgeschirmt in einem Gehäuse angeordnet ist.

Eine dritte bevorzugte Ausführungsform des erfindungsgemässen Sensorsystems ist dadurch gekennzeichnet, dass zwischen Kopfteil und Messteil ein die beiden verbindendes Referenzteil vorgesehen ist, welches eine gegen Flüssigkeit abgedichtete Kammer aufweist, welche ein rotierbar gelagertes Referenzorgan und die genannte sensitive Flüssigkeit enthält. Mittels des Referenzteils wird die Genauigkeit der Messung erhöht und die Einflüsse von Temperaturänderungen auf das Messresultat werden reduziert.

Vorteilhafte Weiterbildungen der zweiten und/oder dritten bevorzugten Ausführungsform sind in den Ansprüchen 10 bis 20 beansprucht.

Im Folgenden wird die Erfindung anhand eines Ausführungsbeispiels und der Zeichnungen näher erläutert; es zeigt:
- Fig. 1, 2: je eine perspektivische Darstellung eines ersten Ausführungsbeispiels des teilweise aufgeschnittenen Sensors eines erfindungsgemässen Sensorsystems,
- Fig. 3: einen Querschnitt durch den Sensor von Fig. 1, 2;
- Fig. 4: ein Blockschema des Bediengeräts des erfindungsgemässen Sensorsystems;
- Fig. 5: eine perspektivische Darstellung eines zweiten Ausführungsbeispiels des teilweise aufgeschnittenen Sensors eines erfindungsgemässen Sensorsystems; und
- Fig. 6: eine perspektivische Ansicht des Sensors von Fig. 5 im geschlossenen Zustand.

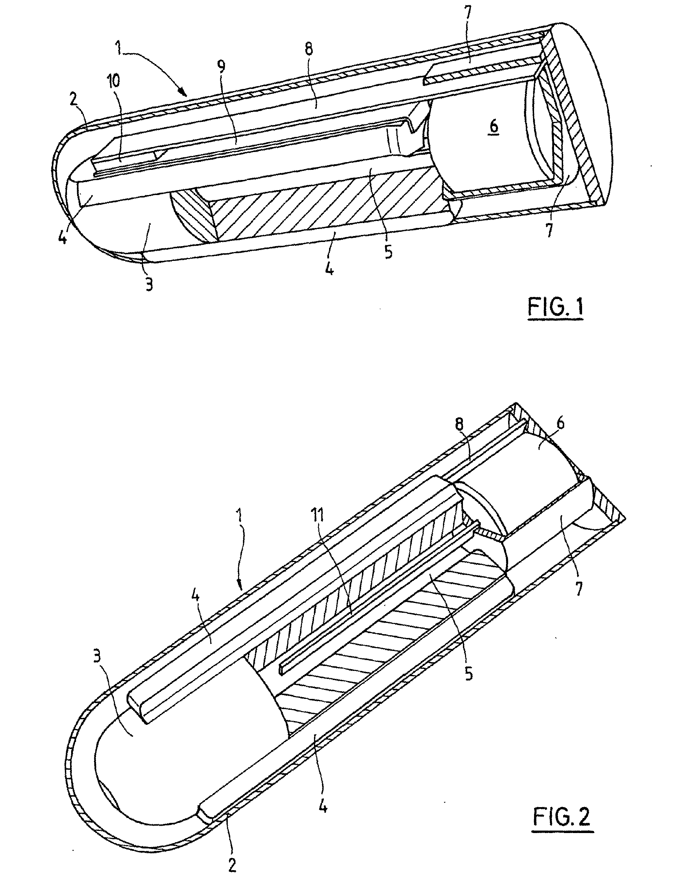

Der in den Figuren 1 bis 3 dargestellte erste Ausführungsbeispiel des Sensors 1 hat die Form einer länglichen Ampulle mit den ungefähren Dimensionen von 2 mm Durchmesser und 8 mm Länge, wobei diese Angaben in weiten Grenzen variabel sind. Der Mantel 2 des Sensors 1 ist durch eine semipermeable Wand aus Cellulose gebildet, durch welche Glukose in die Ampulle eindringen kann. Der grösste Teil des Innenraums des Sensors 1 wird von einem zylindrischen Kunststoffteil 3 eingenommen, das durch mehrere an seinem Mantel abstehende Rippen 4 im Sensor 1 zentriert ist und eine axiale Bohrung 5 aufweist. Das Kunststoffteil, das beispielsweise ein aus Polycarbonat hergestelltes Spritzgussteil ist, dient einerseits als Träger für ein weiter unten beschriebenes Schwingorgan und anderseits zur Verkleinerung des Flüssigkeitsvolumens im Sensor 1. Der Sensor ist im betriebsbereiten Zustand mit einer sensitiven Flüssigkeit mit hohem Molekulargewicht, beispielsweise Dextran und ConA gefüllt.

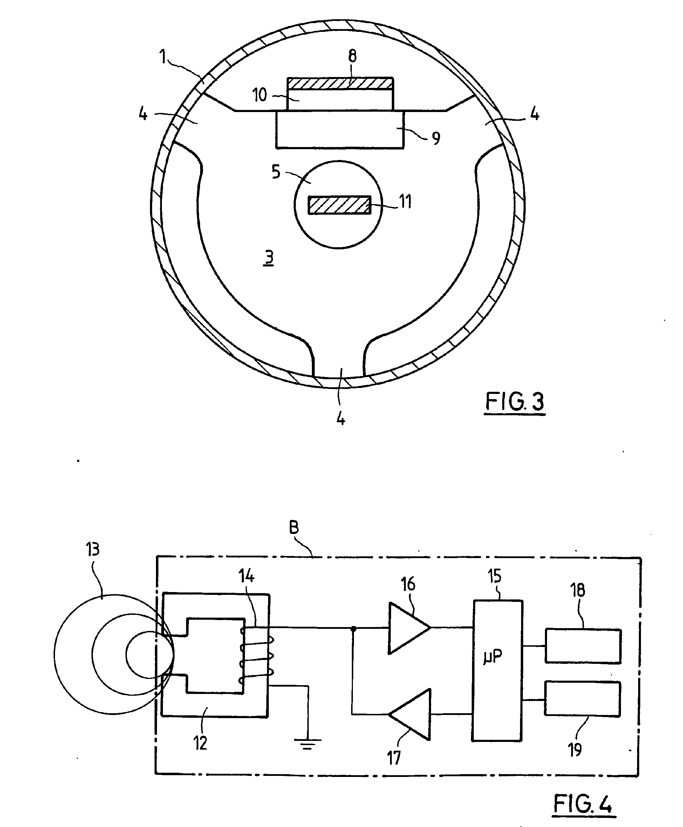

Im Anschluss an das in den Fig. 1 und 2 rechte Ende des Kunststoffteils 3 ist im Sensor 1 ein Permanentmagnet 6 angeordnet, der zur Vermeidung von Korrosion mit einer Kunststoffummantelung 7 aus Polycarbonat überspritzt ist. In den Fig. 1 und 2 sind die Kunststoffummantelung 7, der Mantel 2 des Sensors 1 und das Kunststoffteil 3 teilweise aufgeschnitten, um einen Blick ins Innere des Kunststoffteils 3 zu ermöglichen. Die Kunststoffummantelung 7 dient als Träger für einen Biegebalken 8 aus beispielsweise Aluminiumoxidkeramik, der sich entlang des Kunststoffteils 3 erstreckt. Das Kunststoffteil 3 ist im Bereich des Biegebalkens 8 abgeflacht (Fig. 3) und trägt hier ein Basissubstrat 9 in Form eines dünnen Streifens. Zwischen dem freien Ende des Biegebalkens 8 und dem Basissubstrat 9 ist ein Distanzelement 10 vorgesehen, dessen Dicke so gewählt ist, dass eine genügend grosse Schwingungsamplitude des Biegebalkens von etwa 100 µm möglich ist.

Biegebalken 8, Basissubstrat 9 und Distanzelement 10 bestehen aus dem gleichen Material und sind durch aufeinander Schichten von Laminaten und anschliessendes Verpacken hergestellt. Die Kunststoffummantelung 7 trägt an ihrer dem Kunststoffteil 3 zugewandten Stirnfläche einen schmalen., langgestreckten Flügel 11, der in die Bohrung 5 des Kunststoffteils 3 ragt. Wenn der Permanentmagnet 6 von einem externen oszillierenden Magnetfeld erregt wird, wird er in Vibrationen versetzt und mit der Vibration des Magneten 6 vibrieren auch die Kunststoffummantelung 7, der Biegebalken 8 und der Flügel 11. Diese Vibrationen haben zur Folge, dass die im Sensor 1 vorhandene sensitive Flüssigkeit mit der in den Sensor 1 eingedrungenen Glukose vermischt wird. Dabei ist die Vibration des Flügels 11 von grosser Bedeutung für eine schnelle Messung, weil sie den Fluss im Sensor 1 simuliert und für eine homogene Glukosekonzentration im Sensor sorgt.

Die Frequenz des den Magneten 6 erregenden Magnetfeldes ist so gewählt, dass dieser mit einer Frequenz im Bereich zwischen 100 und 300 Hz vibriert. Biegebalken 8 und Flügel 11 vibrieren mit der gleichen Frequenz, wobei die Schwingungsamplitude etwa 100 µm oder 0.1 mm beträgt. Nach der Durchmischung von sensitiver Flüssigkeit und Glukose wird das Magnetfeld abgeschaltet und die Abklingzeit der Vibration gemessen, was anhand des von dem mit dem Biegebalken 8 mit schwingenden Magneten 6 erzeugten Magnetfeldes erfolgt.

Die Viskositätsänderung von Dextran und ConA in einer physiologisch salinen Lösung in Funktion der Glukose-Konzentration ist in R. Ehwald et al., "Viscosimetric affinity assay", Anal Biochem 234,1 (1996) und U. Beyer, "Recording of subcutaneous glucose dynamics by a viscosimetric affinity sensor", Diabetologia 44, 416 (2001) beschrieben. Die dort beschriebene Lösung basiert auf der Zirkulation der sensitiven Flüssigkeit durch ein aus mehreren Komponenten bestehendes System. Beim erfindungsgemässen System wird die Viskosität direkt im Volumen der im Sensor 1 eingeschlossenen sensitiven Flüssigkeit gemessen, wobei der Sensor in Längsrichtung senkrecht zur Körperoberfläche so unter die Haut implantiert wird, dass das in den Fig. 1 und 2 flache rechte Ende des Sensors 1 etwa 2 mm unterhalb der Haut liegt. Die Implantation erfolgt beispielsweise in Gürtelhöhe mit einer Injektionsnadel.

Fig. 4 zeigt ein Blockschema des mit dem Bezugszeichen B bezeichneten Bediengeräts. Dieses enthält insbesondere einen Magneten 12 zur Erzeugung eines Magnetfeldes 13 für die Erregung des Magneten 6 in der Ampulle 1 (Fig. 1) und eine Spule 14 für die Erregung des Magneten 12, welche gleichzeitig auch als Magnetfeldsensor für die Detektion des vom Magneten 6 im Sensor 1 erzeugten Magnetfeldes dienen, und einen Mikroprozessor 15. Die Spule 14 ist einerseits mit einem Empfangsverstärker 16 und andererseits mit einem Sendeverstärker 17 verbunden, deren Aus- bzw. Eingang an den Mikroprozessor 15 geführt ist. Der Mikroprozessor 15 ist ausserdem mit einer Anzeige 18 für den aktuell gemessenen Glukosewert und mit einem Speicher 19 für die Speicherung der Glukosewerte verbunden. Ausserdem enthält das Bediengerät B eine nicht eingezeichnete Stromversorgung. Optional kann ein zusätzlicher Magnetfeldsensor, beispielsweise ein Hall-Sensor, für die genaue Positionierung (Normalisierung) des Bediengeräts B relativ zum Sensor 1 vorgesehen sein.

Eine andere mögliche Lösung für die Funktionen Anregung und Detektion des Bediengeräts B beruht auf einem rotierenden Dipol, wobei ein Harddisk-Motor mit zwei Permanentmagneten die Schwingungen des Biegebalkens 8 von aussen anregt und durch Analyse der Motordämpfung der Gütefaktor des Oszillators (Biegebalken 8 plus Magnet 6) ermittelt wird.

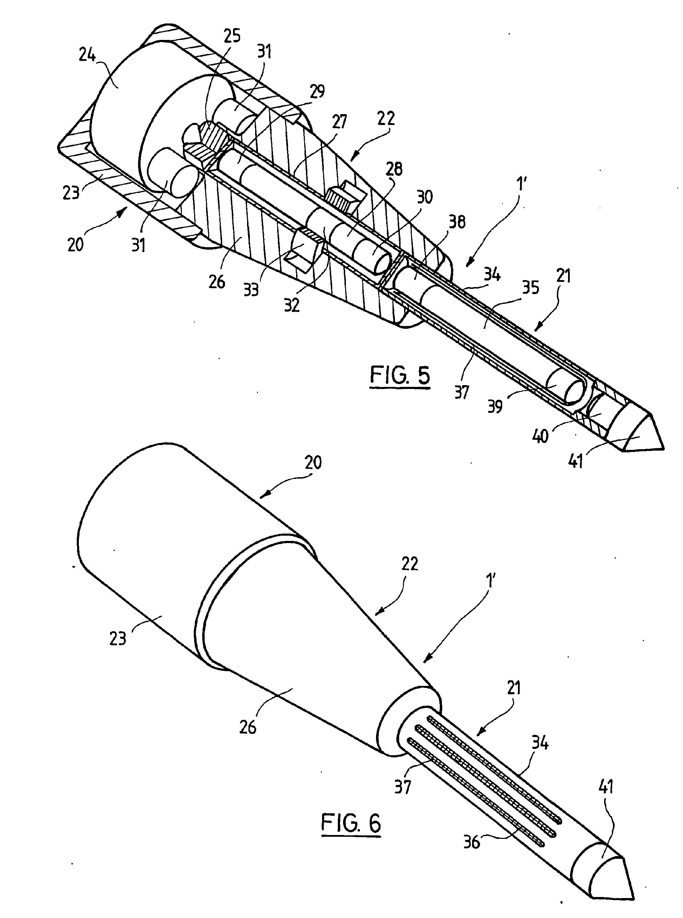

Der in den Figuren 5 und 6 dargestellte Sensor 1' hat ebenfalls die Form einer länglichen Ampulle; er unterscheidet sich von dem in den Figuren 1 bis 3 dargestellten Sensor 1 im wesentlichen durch die Methode der Messung der Viskosität des aus der sensitiven Flüssigkeit und der Glukose bestehenden Gemisches. Während die Viskositätsmessung beim Sensor 1 anhand des Schwingverhaltens eines Schwingorgans erfolgt, erfolgt sie beim zweiten Sensor 1' anhand des Rotationsverhaltens eines Messorgans. Dabei wird das Rotationsverhalten des Messorgans anhand von dessen Abklingverhalten nach Abschalten des Magneten analysiert. Das Messergebnis wird jedoch genauer, wenn zwei Messorgane verwendet werden, von denen das eine in dem aus sensitiver Flüssigkeit und Glukose bestehendem Gemisch und das andere in einer Referenzflüssigkeit rotiert. Vorzugsweise ist die Referenzflüssigkeit durch sensitive Flüssigkeit gebildet.

Gemäss den Figuren 5 und 6 hat der Sensor 1' eine rotationssymmetrische Form und besteht aus einem zylindrischen Kopfteil 20, einem zylindrischen Messteil 21 von geringerem Durchmesser als das Kopfteil 20, und einem Kopfteil 20 und Messteil 21 verbindenden konischen Referenzteil 22. Das Kopfteil 20 hat einen ungefähren Durchmesser von 2.5 mm und eine ungefähre Länge von 3 mm, das Messteil 21 hat einen ungefähren Durchmesser von 0.6 mm und eine ungefähre Länge von 6 mm, und das Referenzteil 22 hat ebenfalls eine ungefähre Länge von etwa 6 mm. Das Kopfteil 20 besteht aus einem luftdichten Gehäuse 23, in welchem ein Antriebsmagnet 24 gelagert ist. Der Antriebsmagnet 16 ist auf zwei Lagern 25 mechanisch abgestützt, von denen aus Fig. 5 nur das im Referenzteil 22 gelagerte vordere Lager ersichtlich ist. Das durch den Antriebsmagneten 24 verdeckte hintere der Lager 25 ist am Gehäuse 23 gelagert.

Das Referenzteil 22 umfasst ein kegelstumpfförmiges Gehäuse 26, welches eine axiale Bohrung aufweist, in der eine luftdichte, zylindrische Referenzkammer 27 angeordnet ist. Das kegelstumpfförmiger Gehäuse 26 ist an seinem dickeren Ende mit dem Kopfteil 20 und an seinem dünneren Ende mit dem Messteil 21 verbunden. In der Referenzkammer 27 befindet sich eine Referenzflüssigkeit, die vorzugsweise durch die in der Beschreibung der Figuren 1 bis 3 genannte sensitive Flüssigkeit mit hohem Molekulargewicht gebildet ist. Ausserdem ist in der Referenzkammer 27 ein zylindrisches Referenzorgan 28 drehbar gelagert.

Das Referenzorgan 28 trägt an seinen Enden je ein magnetisches Endteil 29 und 30, von denen das Endteil 29 eine magnetische Kopplung mit dem Antriebsmagneten 24 und zwei vom diesem abstehenden Permanentmagneten 31 und das Endteil 30 eine magnetische Kopplung mit dem Messteil 21 bildet. Zwischen den beiden magnetischen Endteilen 29 und 30 trägt das Referenzorgan 28 einen weiteren Permanentmagneten 32, der sich im Niveau eines im Gehäuse 26 angeordneten und die Referenzkammer 27 umgreifenden Ringmagneten 33 befindet. Ringmagnet 33 und Permanentmagnet 32 dienen zur Stabilisierung des Referenzorgans 28 in seiner Rotationsachse. Diese Stabilisierung kann auch durch eine mechanische Lagerung der Achse erreicht werden.

Das Messteil 21 umfasst ein zylindrisches Gehäuse 34, welches an seinem einen Ende im Referenzteil 22 befestigt ist und an seinem anderen Ende ein Abschlussteil 22 trägt. Das Gehäuse 34 bildet eine Messkammer, in der sich die genannte sensitive Flüssigkeit befindet, und in der ausserdem ein zylindrisches Messorgan 35 drehbar gelagert ist. Der Mantel des Gehäuses 34 ist mit länglichen Fenstern 36 versehen und innen mit einer semipermeablen, aus Cellulose gebildeten Membran 37 ausgekleidet, durch welche Glukose in die Messkammer eindringen kann. Die Rotation des Messorgans 35 hat zur Folge, dass die in der Messkammer vorhandene sensitive Flüssigkeit mit der in diese eingedrungenen Glukose vermischt wird, was zu homogenen Glukosekonzentration in der Messkammer führt.

Das Messorgan 35 trägt an seinen Enden je ein magnetisches Endteil 38 und 39, von denen das dem Referenzorgan 28 benachbarte Endteil 38 zur magnetischen Kopplung mit dem Referenzorgan 28 und damit zum Antrieb des Messorgans 35 dient. Das andere Endteil 39 bildet eine magnetische Kopplung mit einem Permanentmagneten 40, der am freien Ende des Gehäuses 34 fixiert ist, und dient zur Stabilisierung des Messorgans 35 in seiner Rotationsachse. Mit dem Bezugszeichen 41 ist ein kegelförmiges Abschlussteil des Messteils 21 des Sensors bezeichnet.

Das Bediengerät für das in den Fig. 5 und 6 dargestellte zweite Ausführungsbeispiel des Sensors ist im Wesentlichen gleich aufgebaut wie das in Fig. 4 dargestellte Bediengerät B und unterscheidet sich von diesem hauptsächlich dadurch, dass es mehrere Spulen 14 für die Erzeugung eines Drehfeldes enthält, welches den Antriebsmagneten 24 in Rotation versetzt. Analog weist das Bediengerät mehrere Magnetfeldsensoren auf, welche die Rotation des Antriebsmagneten 24 nach dem Abschalten des magnetischen Drehfeldes messen.

Der Antriebsmagnet 24 treibt über die Magneten 31 und 29 das Referenzorgan 28 an und dieses über die Magneten 30 und 38 das Messorgan 35. Das Messorgan 35 und das Referenzorgan 28 rotieren in den Gehäusen 27 und 34, die beide die gleiche sensitive Flüssigkeit von hohem Molekulargewicht enthalten. Das Gehäuse 27 mit dem Referenzorgan 28 ist luftdicht abgedichtet und das Gehäuse 34 mit dem Messorgan 35 ist mit der semipermeablen Membran 37 abgedichtet, durch welche Glukose in die Messkammer eindringen kann. Die magnetische Kopplung zwischen dem Referenzorgan 28 (Permanentmagnet 30) und dem Messorgan 35 (Permanentmagnet 38) ist so ausgelegt, dass das Messorgan 35 nur bis zu einer kritischen Rotationsfrequenz mitdreht.

Oberhalb dieser kritischen Frequenz misst das System anhand des Abklingens der Rotation des Antriebsmagneten 24 bei Abschaltung des magnetischen Drehfeldes die Viskosität der Flüssigkeit im Gehäuse 27, und diese Flüssigkeit ist ausschliesslich die genannte sensitive Flüssigkeit. Unterhalb der kritischen Frequenz ist das Abklingen der Rotation des Antriebsmagneten 24 bei Abschaltung des magnetischen Drehfeldes durch die Viskosität des Flüssigkeitsgemisches aus sensitiver Flüssigkeit und Glukose in der Messkammer (Gehäuse 34) bestimmt. Die anhand dieser Informationen bestimmte Glukosekonzentration ist von der Temperatur unabhängig, was einen wesentlichen Vorteil gegenüber einem System ohne Referenzmessung darstellt.

Wenn dieser Vorteil nicht gewünscht wird oder nicht wesentlich ist, kann der in den Figuren 5 und 6 dargestellte Sensor durch Weglassen des Referenzteils 22 vereinfacht werden. Der Antriebsmagnet würde in diesem Fall über die Permanentmagnete 31 und 38 das Messorgan 35 direkt antreiben.

Der in den Figuren 1 bis 3 dargestellte Sensor.1 kann durch relativ einfache Modifikation für die Anwendung als Holtersystem adaptiert werden, bei welchem der Glukosegehalt unter ärztlicher Aufsicht und über einen Zeitraum von mehreren Tagen kontinuierlich überwacht wird. Bei dieser Anwendung wird das den Magneten 6 im Sensor 1 erregende Magnetfeld nicht von einem externen Magneten 12 sondern durch eine im Inneren des Sensors 1 angeordnete Stromspule erzeugt, von welcher zwei dünne elektrische Drähte durch die Haut des Patienten nach aussen zum Bediengerät geführt sind. Die genannte Stromspule ist vorzugsweise im Bereich des Distanzelements 10 (Fig. 1) angeordnet. Um einen ausreichenden Magnetfluss von der Stromspule zum Magneten 6 zu gewährleisten, bestehen Biegebalken 8 und Basissubstrat 9 aus weichmagnetischem Material. Gleiches gilt für den in den Figuren 5 und 6 dargestellten Sensor 1', wo ebenfalls im Inneren des Gehäuses 23 eine Stromspule für die Erregung des Antriebsmagneten 24 angeordnet werden könnte.

## Patentansprüche

1. Sensorsystem für die Bestimmung der Glukose-Konzentration im Blut, mit einem implantierbaren Sensor (1, 1') und einem diesem zugeordneten Bediengerät (B), wobei der Sensor (1, 1') die Form einer Ampulle aufweist, in welcher eine sensitive Flüssigkeit eingeschlossen ist und in welche Glukose eindringen kann wobei der Sensor (1,1') eine Messung der Viskosität des aus der sensitiven Flüssigkeit und der Glukose bestehenden Gemisches vornimmt, und wobei das Bediengerät (B) durch ein aussen auf der Haut zu tragendes, portables Gerät gebildet ist, wobei die Steuerung der Messung und deren Auswertung durch das Bediengerät (B) erfolgt, **dadurch gekennzeichnet dass** die Messung der Viskosität anhand des Schwingverhaltens eines im Sensor (1) angeordneten Schwingorgans (8) erfolgt, welches durch ein oszillierendes Magnetfeld zu Schwingungen anregbar ist, und dass das Schwingverhalten des Schwingorgans (8) anhand von dessen Abklingverhalten nach Abschalten des Magneten (6) analysiert wird, wobei das Schwingorgan (8) selbst ein Magnetfeld erzeugt, welches vom Bediengerät gemessen wird.

2. Sensorsystem nach Anspruch 1, **dadurch gekennzeichnet, dass** durch das Schwingorgan zusätzlich eine Homogenisierung der Flüssigkeit im Sensor (1) erfolgt.

3. Sensorsystem nach Anspruch 2, **dadurch gekennzeichnet, dass** das Schwingorgan (8) formschlüssig mit dem Magneten (6) verbunden und durch einen Biegebalken gebildet ist.

4. Sensorsystem nach Anspruch 3, **dadurch gekennzeichnet, dass** der Magnet (6) an einem der beiden Enden des Biegebalkens angebracht und durch ein Magnetfeld (13) in Schwingungen versetzbar ist.

5. Sensorsystem nach Anspruch 4, **dadurch gekennzeichnet, dass** das genannte Magnetfeld (13) durch eine im Bediengerät (B) vorgesehene elektromagnetische Anordnung oder durch eine im Sensor (1) vorgesehene elektrische Spule erzeugt wird.

6. Sensorsystem nach einem der Ansprüche 1 bis 5, **dadurch gekennzeichnet, dass** der Sensor (1) eine das Eindringen von Glukose ermöglichende semipermeable Wand (2) aufweist.

7. Sensorsystem nach einem der Ansprüche 1 bis 6, **gekennzeichnet durch** ein im Sensor (1) angeordnetes, diesen teilweise ausfüllendes und damit das Flüssigkeitsvolumen begrenzendes Kunststoffteil (3), welches als Auflage für das Schwingorgan (8) ausgebildet ist und eine längliche Bohrung (5) aufweist, in welche ein am Magneten (6) angeordneter und zur Durchmischung der Flüssigkeiten vorgesehener Flügel (11) ragt.

8. Sensorsystem nach Anspruch 5, **dadurch gekennzeichnet, dass** die genannte elektro- magnetische Anordnung Mittel zur Erregung des Magneten (6) im Sensor (1) und einen Magnetfeldsensor für das von diesem Magneten erzeugte Magnetfeld enthält.

9. Sensorsystem nach Anspruch 8, **dadurch gekennzeichnet, dass** die genannten Mittel und der genannte Magnetfeldsensor durch einen Magneten (12) und eine diesen erregende Spule (14) sowie einen mit der Spule (14) verbundenen Mikroprozessor (15) gebildet sind.

10. Sensorsystem für die Bestimmung der Glukose-Konzentration im Blut, mit einem implantierbaren Sensor (1, 1') und einem diesem zugeordneten Bediengerät (B), wobei der Sensor (1, 1') die Form einer Ampulle aufweist, in welche eine sensitive Flüssigkeit eingeschlossen ist und in welche Glukose eindringen kann, wobei der Sensor (1,1') eine Messung der Viskosität des aus der sensitiven Flüssigkeit und der Glukose bestehenden Gemisches vornimmt, und wobei das Bediengerät (B) durch ein aussen auf der Haut zu tragendes, portables Gerät gebildet ist, wobei die Steuerung der Messung und deren Auswertung durch das Bediengerät (B) erfolgt, **dadurch gekennzeichnet, dass** die Messung der Viskosität anhand der Rotation eines im Sensor (1') angeordneten Messorgans (35) erfolgt, welches von einem ebenfalls im Sensor (1') angeordneten Antriebsmagneten (24) antreibbar ist und dass die Rotation des Messorgans (35) anhand von deren Abklingverhalten nach Abschalten des Antriebsmagneten (24) analysiert wird.

11. Sensorsystem nach Anspruch 10, **dadurch gekennzeichnet, dass** der Sensor (1') zweistufig aufgebaut ist und ein Kopfteil (20) und ein Messteil (21) aufweist, wobei das Kopfteil (20) den Antriebsmagneten (24) und das Messteil (21) das Messorgan (35) enthält und der Antriebsmagnet (24) gegen Flüssigkeit abgeschirmt und in einem Gehäuse (23) angeordnet ist.

12. Sensorsystem nach Anspruch 11, **dadurch gekennzeichnet, dass** zwischen Kopfteil (20) und Messteil (21) ein die beiden verbindendes Referenzteil (22) vorgesehen ist, welches eine gegen Flüssigkeit abgedichtete Kammer (27) aufweist, welche ein rotierbar gelagertes Referenzorgan (28) und die genannte sensitive Flüssigkeit enthält.

13. Sensorsystem nach Anspruch 11 oder 12, **dadurch gekennzeichnet, dass** das Kopfteil (20) und das Messteil (21) je ein zylindrische Form aufweisen, wobei der Durchmesser des Kopfteils (20) grösser ist als derjenige des Messteils (21).

14. Sensorsystem nach den Ansprüchen 12 und 13, **dadurch gekennzeichnet, dass** das Referenzteil (22) die Form eines Kegelstumpfes aufweist, und dass das Referenzorgan (28) und das Messorgan (35) als längliche Zylinder ausgebildet sind.

15. Sensorsystem nach Anspruch 14, **dadurch gekennzeichnet, dass** das Messteil (21) als längliches Gehäuse (34) ausgebildet ist, welches fensterartige Öffnungen (36) aufweist und innen mit einer das Eindringen von Glukose ermöglichenden semipermeablen Folie (37) ausgekleidet ist.

16. Sensorsystem nach Anspruch 15, **dadurch gekennzeichnet, dass** der Antriebsmagnet (24) durch ein Magnetfeld in Rotation versetzbar ist, welches von einer im Bediengerät (B) vorgesehenen elektromagnetischen Anordnung erzeugt wird.

17. Sensorsystem nach Anspruch 16, **dadurch gekennzeichnet, dass** der Antrieb des Messorgans (35) über magnetische Kopplungen (29,31 ; 30,38) zwischen dem Antriebsmagneten (24) und dem Referenzorgan (28) beziehungsweise zwischen dem Referenzorgan (28) und dem Messorgan (35) erfolgt.

18. Sensorsystem nach Anspruch 17, **dadurch gekennzeichnet, dass** die magnetische Kopplung (30,38) zwischen Referenzorgan (28) und Messorgan (35) so ausgelegt ist, dass das Messorgan (35) nur bis zu einer bestimmten kritischen Rotationsfrequenz mitdreht.

19. Sensorsystem nach Anspruch 18, **dadurch gekennzeichnet, dass** nach Abschalten des Antriebs des Antriebsmagneten (24) das Abklingen von dessen Rotation oberhalb der kritischen Rotationsfrequenz ausschliesslich durch die Viskosität der sensitiven Flüssigkeit in der Kammer (27) des Referenzteils (22) und unterhalb der kritischen Rotationsfrequenz durch die Viskosität des Gemisches aus sensitiver Flüssigkeit und Glukose im Gehäuse (34) des Messteils (21) bestimmt ist.

20. Sensorsystem nach Anspruch 19, **dadurch gekennzeichnet, dass** anhand der beiden Viskositätswerte oberhalb und unterhalb der kritischen Rotationsfrequenz die Bestimmung eines von der Temperatur unabhängigen Werts der Glukosekonzentration erfolgt.

## Claims

1. A sensor system for determining glucose concentration in blood, with an implantable sensor (1, 1') and an operator device (B) associated therewith, wherein the sensor (1,1') has the shape of an ampoule, in which a sensitive liquid is contained and in which glucose may penetrate, wherein the sensor (1, 1') effects a measurement of the viscosity of the mixture comprising the sensitive liquid and glucose, and wherein the operator device (B) is formed by a portable device to be worn outside on the skin, wherein the control of the measurement and its evaluation is effected by way of the operator device (B), **characterized in that** the measurement of the viscosity is made on the basis of the oscillation behaviour of an oscillating member (8) positioned in the sensor (1), which may be excited by an oscillating magnetic field to produce oscillations, and that the oscillation behaviour of the oscillating member (8) is analyzed on the basis of this damping behaviour after turning off the magnet (6), wherein the oscillating member (8) itself produces a magnetic field, which is measured by the operator device.

2. The sensor system according to claim 1, **characterized in that** homogenization of the liquid in the sensor (1) is additionally produced by the oscillating member.

3. The sensor system according to claim 2, **characterized in that** the oscillating member (8) is connected positively to the magnet (6) and is formed by a flexural bar.

4. The sensor system according to claim 3, **characterized in that** the magnet (6) is applied on one of the two ends of the flexural bar and may be excited into oscillations by a magnetic field (13).

5. The sensor system according to claim 4, **characterized in that** the designated magnetic field (13) is produced by an electromagnetic arrangement provided in the operator device (B) or by an electric coil provided in the sensor (1).

6. The sensor system according to any of claims 1 to 5, **characterized in that** the sensor has a semipermeable wall (2) allowing penetration of glucose.

7. The sensor system according to any of claims 1 to 6, **characterized by** a plastic part (3) partly filling the latter and therefore delimiting the volume of liquid, which part is formed as a support for the oscillating member (8) and has a longitudinal bore (5), into which protrudes a blade (11) positioned on the magnet (6) and provided for mixing the liquids.

8. The sensor system according to claim 5, **characterized in that** said electromagnetic arrangement contains means for exciting the magnet (6) in the sensor (1) and a magnetic field sensor for the magnet field produced by this magnet.

9. The sensor system according to claim 8, **characterized in that** said means and said magnetic field sensor are formed by a magnet (12) and a coil (14) exciting the latter as well as by a microprocessor (15) connected to the coil (14).

10. A sensor system for determining glucose concentration in blood, with an implantable sensor (1, 1') and an operator device (B) associated therewith, wherein the sensor (1, 1') has the shape of an ampoule, in which a sensitive liquid is contained and into which glucose may penetrate, wherein the sensor (1, 1') effects a measurement of the viscosity of the mixture comprising the sensitive liquid and glucose and wherein the operator device (B) is formed by a portable device to be worn outside on the skin, wherein control of the measurement and its evaluation is effected by way of the operator device (B), **characterized in that** the measurement of viscosity is made on the basis of the rotation of a measuring member (35) positioned in the sensor (1'), which may be driven by a driving magnet (24) also positioned in the sensor (1') and that the rotation of the measuring member (35) is analyzed on the basis of its damping behaviour after turning off the driving magnet (24).

11. The sensor system according to claim 10, **characterized in that** the sensor (1') is built in two stages and has a head portion (20) and a measuring portion (21), wherein the head portion (20) contains the driving magnets (24) and the measuring portion (21) contains the measuring member (35) and protects the driving magnet (24) from liquid and is positioned in a casing (23).

12. The sensor system according to claim 11, **characterized in that** between the head portion (20) and the measuring portion (21), a reference portion (22) connecting both of them is provided, which has a liquid-proof chamber (27), which contains a rotatably mounted reference member (28) and said sensitive liquid.

13. The sensor system according to claim 11 or 12, **characterized in that** the head portion (20) and the measuring portion (21) each have a cylindrical shape, wherein the diameter of the head portion (20) is larger than that of the measuring portion (21).

14. The sensor system according to claim 12 and 13, **characterized in that** the reference portion (22) has the shape of a frustum, and that the reference member (28) and the measuring member (35) are formed as longitudinal cylinders.

15. The sensor system according to claim 14, **characterized in that** the measuring portion (21) is formed as a longitudinal casing (34), which has window-like apertures (36) and is lined inside with a semi-permeable film (37) allowing penetration of glucose.

16. The sensor system according to claim 15, **characterized in that** the driving magnet (24) may be set into rotation by a magnetic field, which is produced by an electromagnetic arrangement provided in the operator device (B).

17. The sensor system according to claim 16, **characterized in that** the driving of the measuring member (35) is achieved via magnetic couplings (29, 31; 30, 38) between the driving magnet (24) and the reference member (28) between the reference member (28) and the measuring member (35), respectively.

18. The sensor system according to claim 17, **characterized in that** the magnetic coupling (30, 38) is laid out in such a way between the reference member (28) and the measuring member (35) so that the measuring member (35) only jointly rotates up to a certain critical rotation frequency.

19. The sensor system according to claim 18, **characterized in that** after turning off the drive of the driving magnet (24), the damping of its rotation above the critical rotation frequency is exclusively determined by the viscosity of the sensitive liquid in the chamber (27) of the reference portion (22) and below the critical rotation frequency by the viscosity of the mixture of sensitive liquid and glucose in the casing (34) of the measuring portion (21).

20. The sensor system according to claim 19, **characterized in that** the determination of a temperature-independent value of the glucose concentration is achieved on the basis of both viscosity values above and below the critical rotation frequency.

## Revendications

1. Système de capteur pour la détermination du taux de glucose dans le sang, comportant un capteur (1, 1') implantable et un appareil de commande (B) associé à ce dernier, ledit capteur (1, 1') ayant la forme d'une ampoule, dans laquelle est contenu un liquide sensitif et dans laquelle le glucose peut pénétrer, ledit capteur (1, 1') mesurant la viscosité du mélange formé par le liquide sensitif et le glucose, et ledit appareil de commande (B) étant formé par un appareil portable, destiné à être porté extérieurement sur la peau, la commande de la mesure et de son évaluation étant assurée par l'appareil de commande (B), **caractérisé en ce que** la mesure de la viscosité est effectuée à l'appui du comportement oscillatoire d'un oscillateur (8), qui est agencé dans le capteur (1) et qui peut être stimulé en oscillation par un champ magnétique oscillant, et **en ce que** le comportement oscillatoire de l'oscillateur (8) est analysé à l'appui du comportement d'arrêt progressif de ce dernier après la désactivation de l'aimant (6), l'oscillateur (8) générant lui-même un champ magnétique qui est mesuré par l'appareil de commande.

2. Système de capteur selon la revendication 1, **caractérisé en ce que**, sous l'effet de l'oscillateur, il se produit en plus une homogénéisation du liquide dans le capteur (1).

3. Système de capteur selon la revendication 2, **caractérisé en ce que** l'oscillateur (8) est assemblé par emboîtement avec l'aimant (6) et est formé par une barre de flexion.

4. Système de capteur selon la revendication 3, **caractérisé en ce que** l'aimant (6) est agencé sur l'une des deux extrémités de la barre de flexion et peut être amené en oscillation par un champ magnétique (13).

5. Système de capteur selon la revendication 4, **caractérisé en ce que** ledit champ magnétique (13) est généré par un agencement électromagnétique, prévu dans l'appareil de commande (B), ou par une bobine électrique, prévue dans le capteur (1).

6. Système de capteur selon l'une quelconque des revendications 1 à 5, **caractérisé en ce que** le capteur (1) comporte une paroi (2) hémiperméable permettant la pénétration du glucose.

7. Système de capteur selon l'une quelconque des revendications 1 à 6, **caractérisé par** une partie en matière plastique (3), qui est agencée dans le capteur (1), remplit partiellement celui-ci et délimite donc le volume de liquide, et qui est réalisée comme support pour l'oscillateur (8) et comporte une forure (5) oblongue, dans laquelle s'engage une pale (11), agencée sur l'aimant (6) et prévue pour le brassage des liquides.

8. Système de capteur selon la revendication 5, **caractérisé en ce que** ledit agencement électromagnétique contient des moyens d'excitation de l'aimant (6) dans le capteur (1) et un capteur de champ magnétique pour le champ magnétique généré par ledit aimant.

9. Système de capteur selon la revendication 8, **caractérisé en ce que** lesdits moyens et ledit capteur de champ magnétique sont formés par un aimant (12) et une bobine (14) excitant celui-ci, ainsi que par un microprocesseur (15) relié à la bobine (14).

10. Système de capteur pour la détermination du taux de glucose dans le sang, comportant un capteur (1, 1') implantable et un appareil de commande (B) associé à ce dernier, ledit capteur (1, 1') ayant la forme d'une ampoule, dans laquelle est contenu un liquide sensitif et dans laquelle le glucose peut pénétrer, ledit capteur (1, 1') mesurant la viscosité du mélange formé par le liquide sensitif et le glucose, et ledit appareil de commande (B) étant formé par un appareil portable, destiné à être porté extérieurement sur la peau, la commande de la mesure et de son évaluation étant assurée par l'appareil de commande (B), **caractérisé en ce que** la mesure de la viscosité est effectuée à l'appui de la rotation d'un élément de mesure (35), qui est agencé dans le capteur (1') et qui peut être actionné par un aimant de commande (24), également agencé dans le capteur (1'), et **en ce que** la rotation de l'élément de mesure (35) est analysée à l'appui du comportement d'arrêt progressif de ce dernier après la désactivation de l'aimant de commande (24).

11. Système de capteur selon la revendication 10, **caractérisé en ce que** le capteur (1') est réalisé en deux niveaux et comporte une partie de tête (20) et une partie de mesure (21), ladite partie de tête (20) contenant l'aimant de commande (24) et ladite partie de mesure (21) contenant l'élément de mesure (35), et l'aimant de commande (24) est protégé du liquide et est agencé dans un boîtier (23).

12. Système de capteur selon la revendication 11, **caractérisé en ce que** entre la partie de tête (20) et la partie de mesure (21) est prévue une partie de référence (22), qui relie lesdites deux parties et qui comporte une chambre (27), rendue étanche au liquide et contenant un élément de référence (28), monté rotatif, et ledit liquide sensitif.

13. Système de capteur selon la revendication 11 ou 12, **caractérisé en ce que** la partie de tête (20) et la partie de mesure (21) ont chacune une forme cylindrique, le diamètre de la partie de tête (20) étant supérieur au diamètre de la partie de mesure (21).

14. Système de capteur selon les revendications 12 et 13, **caractérisé en ce que** la partie de référence (22) a la forme d'un cône tronqué et **en ce que** l'élément de référence (28) et l'élément de mesure (35) sont réalisés sous forme de cylindres allongés.

15. Système de capteur selon la revendication 14, **caractérisé en ce que** la partie de mesure (21) est réalisée sous la forme d'un boîtier (34) allongé, qui comporte des ouvertures (36) en forme de fenêtres et est revêtu à l'intérieur d'une feuille (37) semiperméable permettant la pénétration du glucose.

16. Système de capteur selon la revendication 15, **caractérisé en ce que** l'aimant de commande (24) peut être entraîné en rotation par un champ magnétique, qui est généré par un agencement électromagnétique prévu dans l'appareil de commande (B).

17. Système de capteur selon la revendication 16, **caractérisé en ce que** l'élément de mesure (35) est actionné par des couplages magnétiques (29, 31 ; 30, 38) entre l'aimant de commande (24) et l'élément de référence (28) et respectivement entre l'élément de référence (28) et l'élément de mesure (35).

18. Système de capteur selon la revendication 17, **caractérisé en ce que** le couplage magnétique (30, 38) entre l'élément de référence (28) et l'élément de mesure (35) est configuré de telle sorte que l'élément de mesure (35) n'est entraîné en rotation que jusqu'à une fréquence de rotation critique déterminée.

19. Système de capteur selon la revendication 18, **caractérisé en ce que**, après la désactivation du système d'entraînement de l'aimant de commande (24), l'arrêt progressif de la rotation de ce dernier au-dessus de la fréquence de rotation critique est déterminé uniquement par la viscosité du liquide sensitif dans la chambre (27) de la partie de référence (22) et, au-dessous de la fréquence de rotation critique, est déterminé par la viscosité du mélange formé par le liquide sensitif et le glucose dans le boîtier (34) de la partie de mesure (21).

20. Système de capteur selon la revendication 19, **caractérisé en ce qu'**une valeur du taux de glucose, indépendante de la température, est déterminée à l'appui des deux valeurs de viscosité au-dessus et au-dessous de la fréquence de rotation critique.
